Europäisches Patentamt

European Patent Office

Office européen des brevets

(19)

(11) **EP 0 846 027 B1**

(12) **EUROPÄISCHE PATENTSCHRIFT**

(45) Veröffentlichungstag und Bekanntmachung des Hinweises auf die Patenterteilung:
**06.10.1999 Patentblatt 1999/40**

(21) Anmeldenummer: 96924819.4

(22) Anmeldetag: 29.06.1996

(51) Int. Cl.[6]: **B01J 19/32**, B01D 3/10

(86) Internationale Anmeldenummer:
**PCT/EP96/02851**

(87) Internationale Veröffentlichungsnummer:
**WO 97/02890 (30.01.1997 Gazette 1997/06)**

(54) **DRUCKVERLUSTARME GEWEBEPACKUNGEN ODER GEWEBEÄHNLICHE PACKUNGEN MIT GEORDNETER STRUKTUR ZUR VERWENDUNG IN STOFFAUSTAUSCHKOLONNEN UND VERFAHREN ZUR REKTIFIKATION UNTER VERWENDUNG DIESER PACKUNGEN**

CLOTH OR CLOTH-LIKE PACKING WHICH IS SUBJECT TO LOW PRESSURE LOSSES AND HAS AN ORDERED STRUCTURE FOR USE IN MATERIAL-EXCHANGE COLUMNS AND RECTIFICATION METHOD USING SUCH PACKING

GARNITURES EN TISSU OU EN MATERIAU SIMILAIRE A FAIBLE PERTE DE PRESSION ET DE STRUCTURE ORDONNEE, DESTINEES A GARNIR DES COLONNES D'ECHANGE DE MATIERE ET PROCEDE DE RECTIFICATION FAISANT APPEL A CES GARNITURES

(84) Benannte Vertragsstaaten:
**CH DE ES FR GB LI NL**

(30) Priorität: **08.07.1995 DE 19524928**
**13.02.1996 DE 19605286**

(43) Veröffentlichungstag der Anmeldung:
**10.06.1998 Patentblatt 1998/24**

(73) Patentinhaber:
**BASF AKTIENGESELLSCHAFT**
**67056 Ludwigshafen (DE)**

(72) Erfinder:
• **BURST, Wolfram**
**D-68199 Mannheim (DE)**
• **HARTMANN, Horst**
**D-67459 Böhl-Iggelheim (DE)**

• **KAISER, Wulf**
**D-67098 Bad Dürkheim (DE)**
• **LAAS, Harald**
**D-67133 Maxdorf (DE)**
• **GRAFEN, Paul**
**D-67273 Weisenheim (DE)**
• **BOCKSTIEGEL, Bernhard**
**D-67354 Römerberg (DE)**
• **BALDENIUS, Kai-Uwe**
**D-67227 Frankenthal (DE)**

(56) Entgegenhaltungen:
EP-A- 0 467 395          WO-A-95/04731
DE-B- 1 088 026          FR-A- 1 575 344
FR-A- 2 212 295          US-A- 2 047 444

• CHEMICAL ENGINEERING, Bd. 63, Nr. 1, Januar 1956, ALBANY, NY, Seiten 246-248, XP002016469 "Gas, Liquid Contact on Wire"

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft Packungselemente für Stoffaustauschkolonnen und daraus gebildete Kolonneneinbauten aus Gewebematerial oder gewebeähnlichem Material, vorzugsweise aus Metallgewebe, mit geordneter Struktur und neuer Geometrie zur Verwendung in einer Stoffaustauschkolonne mit besonders geringem spezifischen Druckverlust sowie deren Anwendung zur Rektifikation von Stoffgemischen.

Bei der destillativen Aufarbeitung von Produktgemischen erzielt man im allgemeinen die besten Ergebnisse bei der sogenannten Gegenstromdestillation auch Rektifikation genannt, d.h. einem Destillationsverfahren bei dem die Flüssigkeit als sogenannter Rücklauf von oben nach unten und der Dampf von unten nach oben in die Kolonne strömt. Dabei kommt es zur Anreicherung der leichterflüchtigen Komponenten im Dampf und der schwerer flüchtigen Komponenten in der Flüssigkeit.

Dieser Stoff- und Warmetransport wird durch in die Kolonne eingebaute Elemente, wie Kolonnenböden, Füllkörper oder geordnete Packungen, die für eine ausreichende Berührungszeit der Phasen und eine hinreichende große Phasengrenzfläche sorgen, intensiviert. Durch diese Kolonneneinbauten, verbunden mit dem abströmenden Rücklauf ergibt sich jedoch in der Kolonne ein Strömungswiderstand für den Dampf, der sogenannte Druckverlust. Der Druckverlust einer Kolonne hängt bei gegebener Kolonnengeometrie, d.h. Durchmesser und Kolonnenhöhe, neben Art und Menge der zu rektifizierenden Verbindungen sehr stark von der Art der Kolonneneinbauten ab.

Für die Auftrennung von Substanzgemischen, die eine hohe Trennleistung erfordern, verwendet man im allgemeinen Rektifizierkolonnen, mit Einbauten aus Metallgewebe mit geordneter Struktur. Diese in regelmäßiger Geometrie systematisch aufgebauten Gewebepackungen mit definierten Durchtrittsbereichen für die Gegenstromphasen zeichnen sich gegenüber allen anderen Einbauten durch höhere Belastbarkeit, eine bessere Trennwirkung und einen geringen spezifischen Druckverlust aus. Sie werden daher bei allen Vakuumrektifikationen eingesetzt, bei denen es wegen der Temperaturempfindlichkeit des zu trennenden Gemisches besonders auf eine Begrenzung des Kolonnendruckverlustes ankommt. Besonders geeignete Kolonnenpackungen sind Metallgewebepackungen vom Typ BX und CY der Firma Sulzer (vgl. Firmenschrift "Trennkolonnen für Destillation und Absorption" der Firma Sulzer) und ähnlich wirksame Metallgewebepackungen anderer Firmen, z.B. Montz-Pak Typ A3 der Montz GmbH und gewebeähnliche Packungen, wie die BSH Typen der Montz GmbH.

Eine schematische Darstellung solcher Kolonnen findet man beispielsweise auf Seite 103 des Lehrbuches "Thermische Trennverfahren" von Klaus Sattler, VCH Verlagsges.mbH, Weinheim (BRD), 1988. Bezüglich näherer Einzelheiten über die Rektifikation von Substanzgemischen verweisen wir auf dieses Lehrbuch von Klaus Sattler, Seiten 101-225, insbesondere 120-160 und 199-214.

Für zahlreiche hochsiedende Gemische ist die thermische Belastbarkeit so gering, daß sich trotz Verwendung der beschriebenen Metallgewebepackungen oder gewebeähnlichen Packungen mit geordneter Struktur und Kopfdrucken in der Kolonne von nur 0,5 bis 1 mbar durch den Druckverlust an den für die erforderliche Trennleistung benötigten Gewebepackungen im Sumpf Temperaturen auf treten würden, die oberhalb des Zersetzungsbereichs der zu trennenden Verbindungen liegt.

Aufgabe der Erfindung war es nun, neue Gewebepackungen oder gewebeähnliche Packungen mit geordneter Struktur zu entwickeln, die bei gleicher Trennleistung einen noch geringeren Druckverlust aufweisen, als alle bislang verfügbaren Kolonneneinbauten, d.h. Gewebepackungen, die es ermöglichen, auch hochsiedende luft- und/oder temperaturempfindliche Substanzgemische, die eine hohe Trennleistung erfordern, ohne Anwendung von alternativen teuren Destillationsverfahren wie Hochvakuumdestillation oder Kurzwegdestillation, mit guten Ausbeuten destillativ zu trennen, bzw. neue Gewebepackungen, die ganz allgemein durch ihren geringen Druckverlust niedrige Sumpftemperaturen und damit eine schonendere Destillation ermöglichen, oder aber die Kapazität der Kolonne vergrößern.

Gegenstand der Erfindung sind nun druckverlustarme Packungselemente für Stoffaustauschkolonneneinbauten vom Innendurchmesser der Kolonne und einer Hohe von 40 bis 300 mm aus formstabilen, geordnet strukturierten und sich berührenden Lagen aus Gewebematerial oder gewebeähnlichem Material mit einer spezifischen Oberfläche von 100 bis 2000 $m^2/m^3$ mit neuer Geometrie, die dadurch gekennzeichnet sind, daß

a) die sich berührenden verformten Lagen aus Gewebematerial oder gewebeähnlichem Material so angeordnet sind, daß sie eine Vielzahl von engen Strömungskanälen, vorzugsweise nahezu dreieckige, nahezu rechtwinklige viereckige oder aber nahezu gleichseitige sechseckige Strömungskanäle bilden, bei denen der Neigungswinkel der Zahnung der einzelnen Gewebelagen der Packung gegen die Kolonnenachse nur 0 bis 14°, vorzugsweise 3 bis 14°, insbesondere 4-6° beträgt und

b) zur Gewährleistung der mechanischen Stabilität der Packungselemente erforderlichenfalls zwischen den Gewebelagen Drähte oder dünne Stäbe in geeigneter, vorzugsweise in waagerechter, Anordnung angebracht sind, oder aber die Gewebelagen an einigen Berührungspunkten fest verbunden sind, um ein Ineinanderrutschen der Gewebelagen zu verhindern.

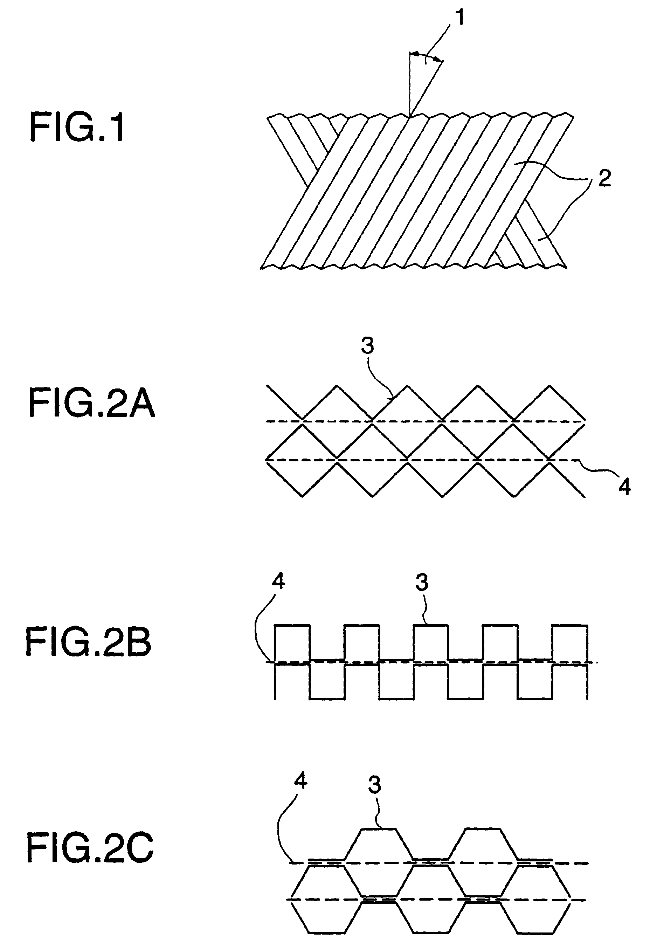

Während also der Neigungswinkel der Zahnung bei den üblichen Gewebepackungen im allgemeinen mindestens 30° beträgt, weisen die erfindungsgemäßen Packungselementen oder scheibenförmigen Kolonnenbauten gebildeten Gewebepackungen einen Neigungswinkel der Zahnung der Gewebelagen gegen die Kolonnenachse von nur 0 bis 14°, vorzugsweise 3 bis 14°, insbesondere 4 bis 6° auf. Der Neigungswinkel der Zahnung der Gewebelagen gegen die Kolonnenachse wird in Figur 1 veranschaulicht. In dieser Figur bedeutet 1 den Neigungswinkel der Zahnung der Gewebelagen gegen die Kolonnenachse und 2 die Gewebelagen.

Wie bei den bekannten Gewebepackungen sind auch bei den erfindungsgemäßen Packungselementen oder in den daraus gebildeten scheibenförmigen Stoffaustauschkolonneneinbauten die einzelnen sich berührenden verformten Lagen aus Gewebematerial oder gewebeähnlichem Material so angeordnet, daß die sich bildenden Strömungskanäle abwechselnd gegensinnig verlaufen, wenn der Neigungswinkel der Zahnung der einzelnen Gewebelagen die Kolonnenachse größer als 0° ist.

Gegenstand der Erfindung sind auch scheibenförmige Stoffaustauschkolonneneinbauten vom jeweiligen Innendurchmesser der Kolonne und einer Höhe von 40 bis 300 mm ,bestehend aus einem oder einer Vielzahl der erfindungsgemäßen Packungselemente aus Gewebematerial oder gewebeähnlichem Material, sowie Kolonneneinbauten, bestehend aus einer Vielzahl von übereinander angeordneten erfindungsgemäßen scheibenförmigen Stoffaustauschkolonneneinbauten, bei denen die scheibenförmigen Kolonneneinbauten jeweils um etwa 90°C zueinander gedreht sind.

Die aus den erfindungsgemäßen Packungselementen oder scheibenförmigen Kolonneneinbauten gebildeten Packungen haben den geringsten spezifischen Druckverlust aller bisher bekannten oder in der Literatur beschriebenen Füllkörper oder Packungen. Der spezifische Druckverlust ist der Druckverlust der Dampfströmung beim Durchströmen der Gewebepackung` deren Höhe genau einer theoretischen Trennstufe entspricht. Bei einem F-Faktor von 1 $\sqrt{Pa}$ liegen die spezifischen Druckverluste der zur Zeit auf dem Markt befindlichen bekannten Packungen mit einer spezifischen Oberfläche von 500 $m^2/m^3$ nach Herstellerangaben im Bereich von 0,09 - 0,11 mbar/$n_{th}$. Die Trennleistung dieser Packungen liegt bei ca. 5 - 7 Trennstufen pro Meter der Packung. Durch die Verwendung der erfindungsgemäßen Gewebepackungen mit senkrechten oder nur wenig zu Kolonnenachse geneigten Strömungskanälen ist es gelungen, bei vergleichbarer Trennleistung den spezifischen Druckverlust um ca. 50 % zu senken.

Hierdurch kann der Anwendungsbereich von Stoffaustauschkolonnen auch auf die Rektifikation von hochsiedenden, luft- und/oder temperaturempfindlichen Substanzen, die eine hohe Trennleistung erfordern, ausgedehnt werden, weil durch die Reduzierung des Druckverlustes die Sumpftemperatur erheblich verringert werden kann.

Es war außerordentlich überraschend, daß die erfindunggemäßen Gewebepackungen oder gewebeähnlichen Packungen mit ihren senkrechten oder nur wenig gegen die Kolonnenachse geneigten, Strömungskanälen bei wesentlich geringerem Druckverlust die gleiche Trennwirkung aufweisen wie die bekannten Gewebepackungen, bei denen der Neigungswinkel der Zahnung der einzelnen Gewebelagen mindestens 30° beträgt (vgl. Gewebepackungen BX, CY, DX und EX der Firma Sulzer Chemtech) bzw. der Neigungswinkel der Wellenkanäle der Metall-Gewebepackungen vom Typ A3 der Firma Montz standardmäßig bei 30 oder 45° liegt, da man bisher davon ausgegangen ist, daß für einen optimalen Stoffaustausch - und damit für optimale Trennwirkung - neben einer guten Verteilung der Flüssigphase auch eine optimale Quervermischung der Dampfphase notwendig ist.

Die einzigen bisher bekannten Packungssysteme aus Metallgewebepackungen mit senkrechten Strömungskanälen sind die sogenannten Kloss- und Neo-Kloss-Packungen der Firma Montz GmbH. Diese Packungen bestehen aus geriffelten oder gewellten Metall-Gewebelagenbändern, die mit Abstandhaltern spiralig zu einem Wickelkörper aufgewickelt sind. Sie enthalten dementsprechend lange senkrechte ringförmige Strömungsspalte, bei denen sich die einzelnen Gewebelagenbänder nicht direkt berühren. Diese Packungssysteme haben zwar einen geringeren Druckverlust als die handelsüblichen Gewebepackungen, jedoch auch eine geringere Trennleistung. Sie haben also keinen geringeren Druckverlust pro Trennstufe, d.h. keinen geringeren spezifischen Druckverlust.

Zur Herstellung der erfindungsgemäßen Packungselemente und Kolonneneinbauten mit neuer Geometrie können alle für die Herstellung von Geweben geeigneten und für die Herstellung von Geweben üblichen Materialien verwendet werden. Als wichtigste seien genannt Metallgewebe aus Edelstählen, wie die entsprechend DIN 1.0330; 1.4000; 1.4301; 1.4401; 1.4404; 1.4435; 1.4439; 1.4571 oder ähnliche, aus Hasteloy C4, aus Aluminium (z.B. DIN 3.0255), aus Kupfer, aus Titan (z.B. DIN 3.7025), aus Monel, aus Kunststoff, wie Polypropylen/Polyacrylnitril-Mischgewebe oder Gewebe aus Glasfasern oder Kohlenstoffasern. Vorteilhaft verwendet man Gewebepackungen mit einer spezifischen Oberfläche von etwa 100 bis 2000 $m^2/m^3$, vorzugsweise 250 bis 1000 $m^2/m^3$. Gewebepackungen, die aus sich berührenden verformten Metallgewebelagen gebildet werden, werden bevorzugt.

Die Verformung der Gewebe kann in an sich bekannter Weise erfolgen. Sie erfolgt z.B. unter Bildung der in Figur 2 dargestellten Formen A), B) und C), in denen die ausgezogenen Linien die einzelnen Gewebelagen in Draufsicht und die gestrichelten Linien die Drähte oder Stäbe zur Gewährleistung der Stabilität in Draufsicht darstellen. In den Figuren 2A, 2B und 2C bedeuten 3 die Gewebelagen und 4 die Drähte oder Stäbe. Besonders vorteilhaft sind Packungen, in denen geriffelte Gewebelagen (entsprechend Figur 2A) die Strömungskanäle bilden.

Zur Gewährleistung der mechanischen Stabilität der Packungselemente werden zwischen den einzelnen Gewebelagen, Drähte oder dünne Stäbe, vorzugsweise Metalldrähte oder dünne Metallstäbe von vorzugsweise etwa 1 mm Durchmesser in geeigneter, vorzugsweise in waagerechter, Anordnung angebracht, die das Ineinanderrutschen der Gewebelagen verhindern sollen. Die mechanische Stabilität der Packungselemente kann aber auch dadurch gewährleistet werden, daß die einzelnen Gewebelagen an einigen Berührungspunkten, beispielsweise durch Verlöten, Verschweißen oder Verkleben fest verbunden werden.

Voraussetzung für eine gute Trennleistung der erfindungsgemäßen Packungen ist - wie bei allen Packungen - eine gute Flüssigkeitsverteilung unterhalb der Zulauf- oder Entnahmestellen. Hierzu sind auf dem Markt sehr gute Flüssigkeitsverteiler mit einer hohen Zahl von Abtropfstellen pro Querschnittsfläche der Kolonne erhältlich. Für eine gute Flüssigkeitsverteilung ist es - insbesondere bei Kolonnen in industriellem Maßstab - auch vorteilhaft, wenn man direkt unterhalb der Flüssigkeitsverteiler ein oder mehrere, vorzugsweise 2 bis 3, scheibenförmige Packungseinbauten mit dem bisher üblichen größeren Neigungswinkel der Zahnung der Gewebelagen anbringt und erst darunter die aus den erfindungsgemäßen Packungselementen gebildeten Kolonneneinbauten anordnet.

Die üblicherweise für Rektifikationen verwendeten Gewebepackungen mit geordneter Struktur sind aus einer der geforderten Trennleistung entsprechenden Anzahl von scheibenförmigen Kolonneneinbauten (Packungslagen) von etwa 15 bis 25 cm Höhe zusammengesetzt. Eine Packungslage besteht im allgemeinen aus einer Vielzahl einzelner entsprechend hoher verformter Gewebelagen. Jede Packungslage wird beim Einbau in die Kolonne um jeweils 90° in bezug auf die vorgehende eingebaut, um eine möglichst gute Stoffverteilung zu gewährleisten.

Auch die erfindungsgemäßen Packungselemente sind oder bilden scheibenförmige Stoffaustauschkolonneneinbauten (Packungslagen), die beim Einbau in die Kolonne um jeweils etwa 90° in bezug auf die vorhergehende Packungslage gedreht werden. Die Höhe der Packungen kann 4 bis 30, vorzugsweise 16 bis 20, insbesondere 16 bis 17 cm, betragen.

Bei einem F-Faktor von etwa 1 $\sqrt{Pa}$ liegen die Druckverluste der zur Zeit auf dem Markt befindlichen bekannten Packungen mit einer spezifischen Oberfläche von 500 $m^2/m^3$ nach Herstellerausgaben in einem Bereich von 0,09 bis 0,11 mbar für eine sogenannte theoretische Trennstufe(nm). Die Trennleistung dieser Packungen liegt bei ca. 5 - 7 Trennstufen pro Meter der Packung. Durch die Verwendung der erfindungsgemäßen Gewebepackungen mit senkrechten oder nur wenig zur Kolonnenachse geneigten Strömungskanälen ist es gelungen, den spezifischen Druckverlust bei vergleichbarer Trennleistung um etwa 50 % zu senken, wodurch sich erhebliche Kapazitätserweiterungen der Kolonne ergeben oder aber Kolonnen mit kleinerem Durchmesser einsetzbar sind.

Gegenstand der Erfindung ist dem entsprechend auch ein Verfahren zur Gewinnung reiner Substanzen aus Substanzgemischen durch Rektifikation dieser Substanzgemische, das dadurch gekennzeichnet ist, daß man hierzu Rektifikationskolonnen verwendet, in denen alle oder zumindest ein Teil der scheibenförmigen Kolonneneinbauten aus den erfindungsgemäßen Packungselementen gebildet werden oder - bei Kolonnen mit kleinem Durchmesser und/oder gut von oben mit Gewebepackungen befüllbaren Kolonnen - solche scheibenförmigen Packungselemente sind.

Durch den geringen spezifischen Druckverlust der neuen Packungen kann der Anwendungsbereich von Stoffaustauschkolonnen auch auf die Rektifikation von Gemischen hochsiedender luft- und/oder temperaturempfindlicher Substanzen, die eine hohe Trennleistung erfordern, im Feinvakuum, d.h. bei Drücken von etwa 0,1 bis 2,5 mbar, ausgedehnt werden, weil durch die Reduzierung des Druckverlustes die Sumpf temperatur erheblich verringert werden kann.

Gegenstand der Erfindung ist dementsprechend auch ein Verfahren zur Gewinnung reiner Substanzen aus Gemischen hochsiedender luft- und/oder temperaturempfindlicher Substanzen, die eine hohe Trennleistung erfordern, durch Rektifikation im Feinvakuum in einer Kolonne enthaltend Metallgewebepackungen mit geordneter Struktur, das dadurch gekennzeichnet ist, daß man die Rektifikation in einer Kolonne ausführt,

a) in der man die Flüssigkeitsverteilung mit Kanalverteilern mit gleich oder mehr als 500 Abstropf stellen pro $m^2$ vornimmt,

b) in der die Kanäle der Flüssigkeitsverteiler im Winkel von etwa 90° zu den Gewebelagen der direkt unter diesen Verteilern befindlichen scheibenförmigen Kolonneneinbauten angeordnet sind,

c) in der unterhalb der Flüssigkeitsverteiler 2 oder mehrere scheibenförmige Kolonneneinbauten mit einer Höhe von nur 20 bis 100 mm eingebaut sind, deren Gewebelagen jeweils um 90° zueinander gedreht sind,

d) in der alle übrigen oder ein Teil der übrigen scheibenförmigen Kolonneneinbauten aus den erfindungsgemäßen Packungslementen gebildet werden,

e) daß die Kolonnen so gestaltet sind, daß während der Rektifikation kein Wärmeaustausch durch die Kolonnenwand stattfinden kann und

f) daß bei luftempfindlichen Substanzen die Kolonne so gestaltet ist, daß praktisch unter Luftausschluß gearbeitet werden kann.

Als Beispiel für ein Gemisch hochsiedender und stark luft- und/oder temperaturempfindlicher Substanzen, die eine hohe Trennleistung erfordern, sei synthetisches Vitamin-E-acetat (VEA) genannt, welches technisch durch Umsetzen von Trimethylhydrochinon mit Phytol oder Isophytol und anschließende Veresterung mit Acetanhydrid hergestellt wird und noch geringe Mengen an gefärbten leichter siedenden und höher siedenden Verunreinigungen enthält. Da VEA in zunehmendem Maße für die menschliche Ernährung bzw. Gesundheitsprophylaxe verwendet wird, werden immer höhere Anforderungen an die Reinheit dieses Produktes gestellt. Die für die Reinigung von Produkten im industriellen Maßstab im allgemeinen sehr vorteilhafte Rektifikation bereitet bei VEA durch dessen hohen Siedepunkt verbunden mit seiner Zersetzlichkeit bei höheren Temperaturen große Probleme. Deshalb werden bisher im wesentlichen Destillationen im Hochvakuum oder gar Molekulardestillationen durchgeführt um VEA bei möglichst niedrigen Temperaturen destillieren zu können.

Da jedoch Destillationen unter Hochvakuum insbesondere aber Molekulardestillationen, bei hohen Reinheiten neben dem Nachteil der geringen Destillationsausbeuten sowohl bezüglich der Investitionskosten als auch hinsichtlich der laufenden Betriebskosten außerordentlich aufwendig und daher sehr teuer sind, haben die erfindungsgemäßen Packungselemente und Kolonneneinbauten eine besondere Bedeutung für die Feinreinigung von VEA, d.h. für den Fall, daß man als Gemisch hochsiedender luft- und/oder temperaturempfindlicher Substanzen, ein mit gefärbten leichter siedenden und höher siedenden Substanzen verunreinigtes VEA rektifiziert.

Gemäß Merkmal a) des diesbezüglichen Anspruchs wird eine Flüssigkeitsverteilung mit Kanalverteilern mit gleich oder mehr als 500 Abtropfstellen beansprucht. Ähnliche auch "Kapillarverteiler" genannte aber runde Verteiler werden von den Firmen Sulzer und Montz vertrieben und werden beispielsweise in EP 512 277 beschrieben. Bekannte Kanalverteiler weisen im allgemeinen nur 50 bis 60 Abtropfstellen pro m² auf.

Die erfindungsgemäße Verwendung der Kanalverteiler bewirkt auf 2 verschiedene Weisen eine Verminderung des sogenannten Druckverlustes. Zum einen bedingen sie eine schnelle äußerst feine Verteilung und damit letztlich eine bessere Nutzung der Packung zum Verteilen des zu trennenden Gemisches und zum anderen eine sehr geringe Berieselungsdichte.

Zur Erreichung einer optimalen Trennleistung ist jedoch nicht nur eine hohe Abtropfstellenzahl sondern auch die Anordnung der Verteiler im Bezug auf die Packungselemente wichtig.

Eine Lage einer Gewebepackung besteht im allgemeinen aus einer Vielzahl üblicherweise einzelner, 170 mm hoher Gewebelagen. Jede Packungslage wird beim Einbau um jeweils 90° im Bezug auf die vorhergehende eingebaut. Die Anordnung der Verteiler erfolgt gemäß Merkmal b) ebenfalls um 90° gedreht in Bezug auf das direkt unter den Verteilern befindliche Packungselement, bzw. die dort befindliche Packungslage.

Die Flüssigkeit breitet sich nun auf einer dieser Gewebelagen in einem bestimmten Winkel aus. Nach einer von dem Ausbreitungswinkel und dem Abstand zweier Abtropfstellen abhängigen Einlauflänge hat sich ein gleichmäßiger Film über einer Gewebelage ausgebildet.

Eine optimale Ausnutzung der Packung, d.h. eine schnellstmögliche Verteilung der Flüssigkeit auf allen Gewebelagen erzielt man, wenn man an dieser Stelle die Packung um 90° dreht.

Unterhalb der Flüssigkeitsverteiler werden gemäß Merkmal c) 2 oder mehrere Packungselemente mit einer Höhe von nur 20 bis 100 mm, vorzugsweise 25 bis 50 mm, insbesondere 35 bis 45 mm eingesetzt, deren Gewebelagen jeweils um 90° zueinander gedreht sind. Durch das Trennen der Packung in Elemente mit geringerer Höhe kann eine schnellstmögliche Verteilung und damit eine optimale Ausnutzung der Packung zum Trennen erzielt werden.

Gemäß Merkmal f) soll die Rektifikation - falls erforderlich - praktisch unter Luftausschluß durchgeführt werden. dies ist bei luftempfindlichen Substanzgemischen, wie bei dem rohen Vitamin-E-acetat besonders wichtig. Die Verwendung von neu entwickelten besonders hochwertigen Dichtungsmaterialien, wie Helicoflex® der Firma Cefilac, zur Abdichtung von Flanschen und/ oder Öffnungen für Geräte zur prozeßüberwachung ist daher unbedingt notwendig. Zur Abdichtung von Flanschen verwendet man mit besonderem Vorteil sogenannte Schweißlippendichtungen, wie sie beispielsweise in den deutschen Patentschriften DE 27 10 859, DE 39 12 478 oder DD 44 07 728 beschrieben sind.

In Feinvakuumrektifikationskolonnen zirkulieren nur geringe Stoffströme. Daher führt jeder Wärmeverlust sofort zu wilder, d.h. unkontrollierter Kondensation an der Kolonnenwand, welche die Trennleistung der Kolonne reduziert. Die gemäß Merkmal e) geforderte Verhinderung eines Wärmeaustausches durch die Kolonnenwand kann am besten durch eine Kombination aus Isolation und Schutzbeheizung der Kolonne gewährleistet werden. Die technische Ausführung einer solchen Schutzbeheizung wird mit Vorteil praktisch auf folgende Weise realisiert: Auf einer ersten Isolierschicht auf dem Kolonnenmantel wird ein Blechmantel angebracht. Dieser Blechmantel wird erneut isoliert. Auf diese Isolierschicht wird dann ein weiterer Blechmantel und die Heizung aufgebracht und diese schließlich nach außen isoliert. Die Regelung der Heizung erfolgt dann derart, daß die Temperaturdifferenz zwischen dem Kolonnenmantel und dem ersten Blechmantel Null beträgt.

Wegen der thermischen Labilität kann man die Rektifikation von VEA bei Verwendung der bekannten Gewebepackungen in Packungskolonnen von maximal 5 m Höhe durchführen.

Bei Verwendung der erfindungsgemäßen Kolonneneinbauten sind durch die Verringerung des Druckabfalls für die destillative Feinreinigung von VEA auch Packungskolonnen mit einer Höhe von mehr als 5 m einsetzbar.

Das nachfolgende Beispiel mit Vergleichsbeispiel soll den großen Vorteil der erfindungsgemäßen Packungen mit neuer Geometrie aufzeigen.

Vergleich des spezifischen Druckverlustes $\Delta p/n_{th}$ einer erfindungsgemäßen Metallgewebepackung mit dem einer bekannten Metallgewebepackung gleicher spezifischer Oberfläche.

Eine Laborkolonne mit einem Durchmesser von 45 mm und einer Länge von 1,8 m wurde jeweils

1) zum Vergleich mit einer handelsüblichen Metallgewebepackung vom Typ Montz A 3-500 mit einer spezifischen Oberfläche von 500 $m^2/m^3$ und einem Neigungswinkel der Wellenkanäle von 30° zur Kolonnenachse und

2) mit einer erfindungsgemäßen Metallgewebepackung mit einer spezifischen Oberfläche von 500 $m^2/m^3$ und einem Neigungswinkel der Zahnung der einzelnen Gewebelagen der Packung gegen die Kolonnenachse von 5° gefüllt.

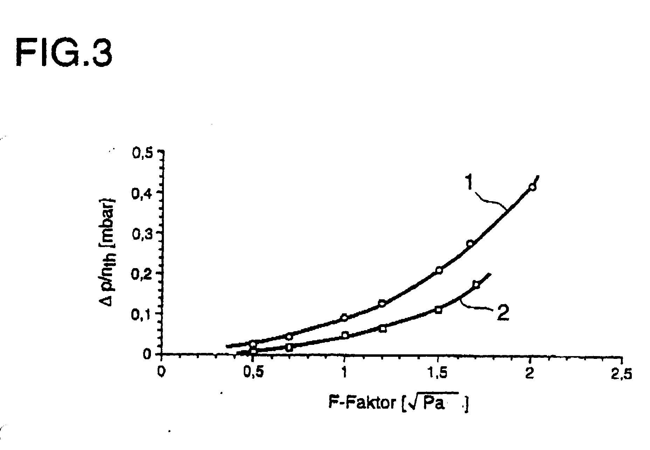

Die Verteilung der Flüssigkeit erfolgte in beiden Fällen mit einer Abtropfstelle, der Kopfdruck betrug 50 mbar. Als Testsystem wurde ein Chlorbenzol/Ethylbenzol-Gemisch verwendet. Es wurde der spezifische Druckverlust ($\Delta p/n_{th}$) bei einem F-Faktor von 0,5 bis etwa 2 bestimmt und die erhaltenen Werte in Figur 3 als Kurven dargestellt.

In Figur 3 stellt die Kurve 1 den Druckverlust für die Gewebepackung vom Typ Montz A3 (Vergleich) und die Kurve 2 den Druckverlust für die erfindungsgemäße Metallgewebepackung dar.

Wie die Kurven zeigen, weist die erfindungsgemäße Packung einen wesentlich geringeren spezifischen Druckverlust auf. Im Belastungsbereich zwischen F = 0,5 und 2 $\sqrt{Pa}$ ist der spezifische Druckverlust nur etwa halb so groß wie der der Gewebepackung vom Typ Montz A 3-500 mit gleicher Trennwirkung.

**Patentansprüche**

1. Druckverlustarme Packungselemente für Stoffaustauschkolonneneinbauten vom Innendurchmesser der Kolonne und einer Höhe von 40 bis 300 mm aus formstabilen geordnet strukturierten und sich berührenden Lagen aus Gewebematerial oder gewebeähnlichem Material mit einer spezifischen Oberfläche von 100 bis 2000 $m^2/m^3$, dadurch gekennzeichnet, daß

   a) die sich berührenden verformten Lagen aus Gewebematerial oder gewebeähnlichem Material so angeordnet sind, daß sie eine Vielzahl von engen Strömungskanälen bilden, bei denen der Neigungswinkel der Zahnung der einzelnen Gewebelagen gegen die Kolonnenachse 0 bis 14° beträgt und

   b) zur Gewährleistung der mechanischen Stabilität der Packungselemente erforderlichenfalls zwischen den Gewebelagen Drähte oder dünne Stäbe in geeigneter Anordnung angebracht sind, oder aber die Gewebelagen an einigen Berührungspunkten fest verbunden sind, um ein Ineinanderrutschen der Gewebelagen zu verhindern.

2. Packungselemente für Stoffaustauschkolonneneinbauten nach Anspruch 1, dadurch gekennzeichnet, daß der Neigungswinkel der Zahnung der einzelnen Gewebelagen der Packungselemente gegen die Kolonnenachse 3 bis 14° beträgt.

3. Packungselemente für Stoffaustauschkolonneneinbauten nach Anspruch 1, dadurch gekennzeichnet, daS die sich berührenden strukturierten Lagen aus Metallgewebe oder gewebeähnlichem Material aus Metall gebildet werden.

4. Packungselemente für Stoffaustauschkolonneneinbauten nach Anspruch 1, dadurch gekennzeichnet, daß zur Gewährleistung der mechanischen Stabilität der Packungselemente zwischen den einzelnen Gewebelagen Drähte oder Stäbe in waagerechter Anordnung angebracht sind.

5. Packungselemente für Stoffaustauschkolonneneinbauten nach Anspruch 1, dadurch gekennzeichnet, daß die sich berührenden verformten Lagen aus Gewebematerial oder gewebeähnlichem Material so angeordnet sind, daß sie nahezu dreieckige, nahezu rechtwinklige viereckige oder aber nahezu gleichseitige sechseckige Strömungskanäle bilden.

6. Packungselemente für Stoffaustauschkolonneneinbauten nach Anspruch 1, dadurch gekennzeichnet, daß die sich berührenden verformten Lagen aus Gewebematerial oder gewebeähnlichem Material so angeordnet sind, daß die durch sie gebildeten Strömungskanäle abwechselnd gegensinnig verlaufen wenn der Neigungswinkel der Zahnung der einzelnen Gewebelagen gegen die Kolonnenachse größer als >0 ist.

7. Scheibenförmige Stoffaustauschkolonneneinbauten vom Innendurchmesser der Kolonne und einer Höhe von 40 bis 300 mm bestehend aus einem oder einer Vielzahl von Packungselementen aus formstabilen geordnet strukturierten und sich berührenden Lagen aus Gewebematerial oder gewebeähnlichem Material gemäß Anspruch 1.

8. Kolonneneinbauten bestehend aus einer Vielzahl von übereinander angeordneten scheibenförmigen Stoffaustauschkolonneneinbauten gemäß Anspruch 7, bei denen die scheibenförmigen Kolonneneinbauten jeweils um etwa 90° zueinander gedreht sind.

9. Verfahren zur Gewinnung reiner Substanzen aus Substanzgemischen durch Rektifikation dieser Substanzgemische, dadurch gekennzeichnet, daß man hierzu Rektifikationskolonnen verwendet, in denen alle oder zumindest ein Teil der scheibenförmigen Kolonneneinbauten aus Packungselementen gemäß Anspruch 1 gebildet werden oder solche sind.

10. Verfahren zur Gewinnung reiner Substanzen aus Gemischen hochsiedender luft- und/oder temperaturempfindlicher Substanzen, die eine hohe Trennleistung erfordern, durch Rektifikation im Feinvakuum in Kolonnen enthaltend Metallgewebepackungen mit geordneter Struktur, dadurch gekennzeichnet, daß man die Rektifikation in einer Kolonne ausführt,

a) in der man die Flüssigkeitsverteilung mit Kanalverteilern mit gleich oder mehr als 500 Abtropfstellen pro m$^2$ vornimmt,

b) in der die Kanäle der Flüssigkeitsverteiler im Winkel von etwa 90° zu den Gewebelagen der direkt unter diesen Verteilern befindlichen scheibenförmigen Kolonneneinbauten angeordnet sind,

c) in der unterhalb der Flüssigkeitsverteiler 2 oder mehrere scheibenförmige Kolonneneinbauten mit einer Höhe von nur 20 bis 100 mm eingebaut sind, deren Gewebelagen jeweils um 90° zueinander gedreht sind,

d) in der alle übrigen oder ein Teil der übrigen scheibenförmigen Kolonneneinbauten aus Packungselementen gemäß Anspruch 1 gebildet werden,

e) daß die Kolonnen so gestaltet sind, daß während der Rektifikation kein Wärmeaustausch durch die Kolonnenwand stattfinden kann und

f) bei luftempfindlichen Substanzen die Kolonne so gestaltet ist, daß praktisch unter Luftausschluß gearbeitet werden kann.

**Claims**

1. Packing elements with a low pressure drop for mass transfer column internals of the internal diameter of the column and of a height of from 40 to 300 mm made of dimensionally stable layers, which have an ordered structure and are in mutual contact, of cloth material or cloth-like material with a specific surface area of from 100 to 2000 m$^2$/m$^3$, wherein

a) the shaped layers of cloth material or cloth-like material which are in mutual contact are arranged so that they form a multiplicity of narrow flow channels in which the angle of inclination of the serration of the individual cloth layers to the column axis is 0 to 14°, and

b) to ensure mechanical stability of the packing elements, if necessary wires or thin rods are fixed in suitable arrangement between the cloth layers, or else the cloth layers are firmly connected at some points of contact, in order to prevent the cloth layers slipping into one another.

2. Packing elements for mass transfer column internals as claimed in claim 1, wherein the angle of inclination of the serration of the individual cloth layers of the packing elements to the column axis is 3 to 14°.

3. Packing elements for mass transfer column internals as claimed in claim 1, wherein the structured layers in mutual contact are formed from metal cloth or cloth-like material made of metal.

4. Packing elements for mass transfer column internals as claimed in claim 1, wherein, to ensure mechanical stability of the packing elements, wires or rods are fixed in horizontal arrangement between the individual cloth layers.

5. Packing elements for mass transfer column internals as claimed in claim 1, wherein the shaped layers of cloth material or cloth-like material which are in mutual contact are arranged so that they form virtually triangular, virtually rectangular or else virtually equilateral hexagonal flow channels.

6. Packing elements for mass transfer column internals as claimed in claim 1, wherein the shaped layers of cloth material or cloth-like material which are in mutual contact are arranged so that the flow channels formed by them alternately run in opposite directions when the angle of inclination of the serration of the individual cloth layers to the column axis is greater than 0.

7. Disk-shaped mass transfer column internals of the internal diameter of the column and of a height of from 40 to 300 mm consisting of one or a multiplicity of packing elements made of dimensionally stable layers of cloth material or clothlike material which have an ordered structure and are in mutual contact, as claimed in claim 1.

8. Column internals consisting of a multiplicity of disk-shaped mass transfer column internals as claimed in claim 7, which are arranged one above the other, where the disk-shaped column internals are rotated by about 90° relative to each other.

9. A process for obtaining pure substances from substance mixtures by rectification of these substance mixtures, which comprises using for this purpose rectification columns in which all or at least part of the disk-shaped column internals are formed from packing elements as claimed in claim 1, or are such.

10. A process for obtaining pure substances from mixtures of high-boiling air- and/or temperature-sensitive substances which require a high separation efficiency, by rectification under medium vacuum in columns containing metal cloth packings with an ordered structure, which comprises carrying out the rectification in a column

    a) in which the liquid distribution is carried out by channel distributors with 500 or more drip points per m$^2$,

    b) in which the channels of the liquid distributors are arranged at an angle of about 90° to the cloth layers of the disk-shaped column internals located immediately below these distributors,

    c) in which two or more disk-shaped column internals which have a height of only from 20 to 100 mm and whose cloth layers are rotated by 90° relative to each other are fitted below the liquid distributors,

    d) in which all other or some of the other disk-shaped column internals are formed from packing elements as claimed in claim 1,

    e) wherein the columns are designed so that no heat exchange can take place through the column wall during the rectification, and

    f) the column is designed in the case of air-sensitive substances so that it can be operated virtually with exclusion of air.

**Revendications**

1. Éléments de garnissage à faible perte de charge, pour installations de colonnes d'échange de matières du diamètre intérieur de la colonne et sur une hauteur de 40 à 300 mm, dans un état structuré, disposés en une forme stable et en contact les uns avec les autres, en matière tissée ou analogue à un tissu, ayant une surface spécifique de 100 à 2000 m$^2$/m$^3$, caractérisés par le fait que

    a) les couches conformées, en contact les unes avec les autres, en matière tissée ou analogue à un tissu, sont agencées de façon à former une multitude de canaux d'écoulement étroits, dans lesquels l'angle d'incidence de la dentelure ou entaille des couches de tissu individuelles est de 0 à 14° par rapport à l'axe de la colonne et
    b) pour assurer la stabilité mécanique des éléments de garnissage, si nécessaire, entre les couches de tissu, des fils ou des tiges minces sont placés dans une disposition appropriée, ou encore les couches de tissu sont liées fermement en certains de leurs points de contact, pour empêcher que les couches de tissu glissent les unes dans les autres.

2. Éléments de garnissage pour installations de colonnes d'échange de matières selon la revendication 1, caractérisés par le fait que l'angle d'incidence de la dentelure ou entaille des couches de tissu individuelles des éléments de garnissage est de 3 à 14° par rapport à l'axe de la colonne.

3. Éléments de garnissage pour installations de colonnes d'échange de matières selon la revendication 1, caractérisés par le fait que les couches structurées contiguës sont formées d'un tissu métallique ou d'un matériau en métal semblable à un tissu.

4. Éléments de garnissage pour installations de colonnes d'échange de matières selon la revendication 1, caractérisés par le fait que, pour assurer la stabilité mécanique des éléments de garnissage, des fils ou des tiges sont disposés horizontalement entre les couches de tissu individuelles.

5. Éléments de garnissage pour installations de colonnes d'échange de matières selon la revendication 1, caractérisés par le fait que les couches conformées contiguës en tissu ou en matériaux semblables à un tissu sont disposées de façon à former des canaux d'écoulement pratiquement triangulaires, pratiquement quadrangulaires orthogonaux, ou encore hexagonaux équilatéraux.

6. Éléments de garnissage pour installations de colonnes d'échange de matières selon la revendication 1, caractérisés par le fait que les couches conformées contiguës sont constituées d'un tissu ou d'un matériau semblable à un tissu, de telle sorte que les canaux d'écoulement formés à travers elles sont orientés de façon inversée alternée lorsque l'angle d'incidence de la dentelure ou entaille des couches de tissu individuelles est > 0 par rapport à l'axe de la colonne.

7. Éléments internes pour colonnes d'échange de matières, en forme de disque, au diamètre intérieur de la colonne et d'une hauteur de 40 à 300 mm, consistant en un ou plusieurs éléments de garnissage formés de couches agencées pour avoir une forme stable, structurées et contiguës, en tissu ou en matériau semblable à du tissu selon la revendication 1.

8. Éléments intérieurs de colonnes consistant en une multitude d'éléments intérieurs de colonnes d'échange de matières en forme de disques empilés les uns sur les autres selon la revendication 7, dans lesquels les éléments intérieurs de colonne en forme de disques sont tournés d'environ 90° les uns par rapport aux autres.

9. Procédé pour l'obtention de substances pures à partir de mélanges de substances par rectification de ces mélanges de substances, caractérisé par le fait qu'on utilise pour ce faire des colonnes de rectification dans lesquelles la totalité ou au moins une partie des éléments intérieurs de colonne en forme de disques sont constitués d'éléments de garnissage selon la revendication 1 ou analogues.

10. Procédé pour l'obtention de substances pures à partir de mélanges de substances à point d'ébullition élevé, sensibles à l'air et/ou à la température, nécessitant un pouvoir de séparation élevé, par rectification sous vide poussé dans des colonnes contenant des garnissages en tissu métallique ayant une structure ordonnée, caractérisé par le fait que la rectification s'effectue dans une colonne

a) dans laquelle on effectue la répartition des fluides avec des répartiteurs sous forme de canaux ayant autant ou plus de 500 points d'écoulement par m$^2$,

b) dans laquelle les canaux des répartiteurs de fluides sont disposés avec un angle d'environ 90° par rapport aux couches de tissu des éléments internes de colonne en forme de disques se trouvant directement en-dessous de ces répartiteurs,

c) dans laquelle sont installés en-dessous des répartiteurs de fluides au moins 2 éléments intérieurs de colonne en forme de disque ayant une hauteur de seulement 20 à 100 mm, dont les couches de tissu sont à chaque fois tournées de 90° les unes par rapport aux autres,

d) dans laquelle tous les autres ou une partie des autres éléments intérieurs de colonne en forme de disques constitués d'éléments de garnissage sont conformés selon la revendication 1,

e) ensuite, les colonnes sont agencées de telle sorte que, pendant la rectification, il ne puisse de produire aucun échange thermique à travers la paroi de la colonne et

f) pour les substances sensibles à l'air, la colonne est agencée de telle sorte qu'on puisse opérer pratiquement en l'absence d'air.

FIG.1

FIG.2A

FIG.2B

FIG.2C

# FIG.3